# EUROPEAN PATENT APPLICATION

(11) **EP 0 610 860 A2**
(43) Date of publication of application: **17.08.1994**
(21) Application number: 94101829.3
(22) Date of filing: 07.02.1994
(51) Int. Cl.: G01N 33/18, G01N 21/64, G01N 21/78, G01N 31/22, C02F 9/00, C02F 1/00, C02F 3/00

(54) **Monitoring hydraulic characteristics of raw and waste water treatment operations**

(30) Priority: 08.02.1993 US 14501
(71) Applicant: NALCO CHEMICAL COMPANY, Naperville Illinois 60563-1198 (US)
(72) Inventor: Avallone, Stanley C., Lockport, Illinois 60441 (US); Eisner, Ian E., Aurora, Illinois 60504 (US)
(74) Representative: Ruschke, Olaf

(57) **Abstract**

A process monitors at least one hydraulic characteristic in a raw or waste water stream and/or system of said raw or waste water treatment process. At least one fluorescent specie(s) that has a maximum excitation wavelength of less than about 400 nm is added to a raw or waste water stream and/or system of said raw or waste water treatment process and at least one sample of water from said raw or waste water stream and/or system of said raw or waste water treatment process is analyzed for at least the presence of the fluorescent specie(s), and the presence of the specie of fluorescent specie(s) in such sample determines at least one hydraulic characteristic of the process.

## Description

### Technical Field of the Invention

The present invention is in the technical field of industrial raw and waste water treatment processes (clarification and/or waste reduction processes) and more particularly in the technical field of monitoring the hydraulic characteristics of such raw and waste water treatment processes so as to increase the efficiency thereof.

### Background of the Invention

The hydraulic characteristics of industrial raw and waste water treatment processes have been monitored by adding to the water systems and/or water streams thereof chemicals such as lithium and visible dyes, and then monitoring such signature chemicals. The monitoring requires analysis of samples taken from the water stream and/or system to determine at least the presence, and often the concentration, of the signature chemical. Lithium analysis cannot normally be done outside of laboratory facilities and requires several days for completion. The delay due to the sample-transport and analysis time diminishes the value of lithium monitoring for raw and waste water treatment processes. The feedback information is inevitably days behind the sampling, and the hydraulic characteristics routinely have changed during the interval. This disadvantageous time delay between sampling and completion of the analysis has been avoided when visible dyes are used as the signature chemical. (Visible dyes, or dyes, as such terminology is used herein, and as understood generally in the chemical field, are colored substances that bring about a significant degree of coloration in other materials when dispersed therein, which coloration characteristic is due to their strong absorption of electromagnetic radiation in the visible region, which extends from about 4000 to about 7000 Angstroms, or from about 400 to about 700 nm.) The presence, and often the concentration, of visible dyes can be determined on-site when a sufficient concentration of such dyes are employed. The primary advantage in using visible dyes is to permit analysis by sight. The color imparted to the water stream and/or system by visible dyes is nonetheless often a major disadvantage. The color gives the treated water stream and/or system a sinister and/or tainted appearance. The color normally persists, and taints the appearance of effluents and/or recycled fractions. The persistent color can even at times diminish the recycling potential of a waste water treatment process. Moreover, the concentration of visible dyes required for their monitoring purposes can add to the organic loading of the water stream and/or system and thus be a detrimental factor to the water treatment process.

It is an object of the present invention to provide a method or process for monitoring one or more of the hydraulic characteristics of raw and waste water treatment processes that can be conducted on-site in a very short time period without adding a visible dye to the raw or waste water stream and/or the system thereof. It is an object of the present invention to provide a water treatment process wherein one or more of the hydraulic characteristics is monitored on-site in a very short time period without adding a visible dye to the raw or waste water stream and/or the system thereof. These and other objects of the present invention are discussed in detail below.

### Disclosure of the Invention

The present invention provides a process for the monitoring of at least one hydraulic characteristic of a raw or waste water treatment process comprising adding to the water of said raw or waste water process a fluorescent specie(s) that has a maximum excitation wavelength of less than about 400 nm, and a raw or waste water treatment process that includes such monitoring step.

### Brief Description of the Drawings:

FIGURE 1 is a plot of the apparent concentrations of a tracer at the primary-pond outfall of an industrial ash pond versus time (in days), from data which was obtained employing the process of the present invention.

FIGURE 2 is a plot of the apparent (uncorrected) concentrations of a tracer at the system outfall of an industrial ash pond versus time (in days), from data which was obtained employing the process of the present invention.

FIGURE 3 is the plots of the percent throughput of an industrial ash pond versus time (in hours) between the primary-pond inlet and primary-pond outfall and then between the primary-pond inlet and system outfall using the apparent (unadjusted) tracer concentration readings, from data which was obtained employing the process of the present invention.

FIGURE 4 is the plot of a percent throughput versus time (in days) between the primary-pond inlet and primary-pond outfall of an industrial ash pond using adjusted tracer concentration readings, from data which was obtained employing the process of the present invention.

FIGURE 5 is the plot of a percent throughput versus time (in days) between the primary-pond inlet and system outfall of an industrial ash pond using adjusted tracer concentration readings, from data which was obtained employing the process of the present invention.

FIGURE 6 is a plot of the apparent tracer concentration versus time (in days) at the primary-pond inlet of an industrial ash pond, from data which was obtained employing the process of the present invention.

### Preferred Embodiments of the Invention

The present invention includes the tracing or tracking of the fluorescent specie(s) in the water stream and/or system of the raw or waste water treatment process. By the terms "tracing" and "tracking" is meant herein, unless expressly indicated otherwise, the determination of at least the presence of the fluorescent specie(s) in at least one sample of the water stream and/or system, and at times the concentration of the fluorescent specie(s) in such sample. Such tracing can be conducted on a singular, intermittent, semi-continuous or continuous basis, and preferably is conducted on-site (at the site of the raw or waste water treatment plant).

The present invention comprises the monitoring of at least one hydraulic characteristic of the raw or waste water treatment process by employing the information provided by the tracking of the fluorescent specie(s). The hydraulic characteristic monitored by the process of the present invention may be any hydraulic characteristic of the raw or waste water treatment process, and/or the raw or waste water stream and/or system, concerning and/or effected by the movement of water within the water stream and/or system, including but not limited to throughput times, waste water stream routes, mixtures of multiple streams, channelling, rise rate, carry over, cross contamination, leaks between systems, flow paths and rates in distribution piping and sewers, and the like.

By "monitoring a hydraulic characteristic" is meant herein, unless expressly indicated otherwise, a determination of a value of a hydraulic characteristic, which value may be a relative value, a substantially quantitative value, or an approximate quantitative value. The monitoring of the process of the present invention often combines at least some of the information provided by the tracking of the fluorescent specie(s) with at least some of the information available concerning the addition of the fluorescent specie(s) to the raw or waste water stream and/or system.

The basic monitoring of a water stream and/or system of a raw or waste water treatment process may involve the addition of a slug of the fluorescent specie(s) to the water stream and/or system at a known point in time, a plurality of water samplings at one or more sites downstream of the addition site at known point in times and the analysis of a plurality of the water samples for at least the presence of the fluorescent specie(s). The appearance and disappearance of the fluorescent specie(s), or preferably the rise and drop in the fluorescent specie(s) concentration, at the sampling site(s) reveals the throughput time for a water-soluble specie(s) between the introduction and sampling site(s). Such information discloses the exposure time of any water soluble species to treatment between two sites that have been monitored, and thus permits a more accurate determination of the degree of any time-dependent treatment of such water soluble specie(s). Such information not only permits a more accurate and efficient treatment plan for normal influent solutes, but also for upsets in the inlet water quality. The information provided by the monitoring is directly related to the hydraulic characteristic of primary importance.

In some raw and/or waste water treatment processes, it is not the throughput time at a given time period that is of primary importance but instead possibly the average daily throughput time, or the duration and level of the maximum throughput time, or cyclic variations in throughput time, or the total daily throughput, all of which and more can be provided by the use of the process of the present invention, preferably by the tracking of the fluorescent specie(s) at one or more sites downstream of the addition point, which advantageously can at times be by intermittent sampling(s) of the water followed by analyses of the sample(s) for the fluorescent specie(s). In these and other variations of monitoring hydraulic characteristics using a fluorescent specie(s), the hydraulic characteristic may be evaluated from the concentration of the fluorescent specie(s), the change in concentration of the fluorescent specie(s), or the mere detection of the fluorescent specie(s) without determining its concentration, for instance when the passing of an intermittent fluorescent species slug is determined by merely its detection at a given site.

Other basic hydraulic characteristic monitorings may instead depend on the change in the concentration of the fluorescent specie(s) at a given site, which is also, or close to, its addition site. Such hydraulic characteristics include holding times which, unlike evaporation rates and the like, cannot generally be determined solely by observed volumetric changes.

Other basic hydraulic characteristic monitorings may depend on the concentration of the fluorescent specie(s) at multiple sites, which typically would be downstream of the addition point. Such hydraulic characteristics include flow patterns or routes, particularly when multiple routes are available to a waste water stream and the route(s) taken cannot be readily discerned visually.

Hydraulic characteristics such as flow patterns among a plurality of contiguous and/or merging streams may also be monitored by the process of the present invention by the relative decrease in the concentration of the fluorescent specie(s) upon dilution, which dilution may be a function of stream merging or leakage between streams or cross-contamination, or such hydraulic characteristics may be evaluated by using a plurality of fluorescent species each of which are introduced at different sites, which may be sites along separate streams.

The industrial raw and waste water streams and/or systems for which the present invention may be employed may be used for, or derived from, most any industry that generates a waste water stream and/or system and/or that purifies a raw water stream and/or system for makeup use, including without limitation the pulping and papermaking industries, the steel industry, the metal working industries, the food processing industries, the mining industries, the automotive industry, the textile industry, utilities, chemical processing industries, manufacturing industries, and spray paint industries. The solids and/or solutes within the waters of these streams and/or systems may be substantially or mainly organic, or substantially or mainly inorganic, or a mixture of both organic and inorganic materials. In contrast, the process of the present invention would generally not be applicable to a waste water stream and/or system of a raw sewage treatment plant due to the high solids loadings of such a stream and/or system.

A raw or waste water stream or system may contain dissolved solids or dissolved gases, or it may be a slurry (dilute or concentrated), or it may be a slurry containing dissolved solids and/or gases. A raw or waste water stream and/or system may also contain liquids other than water, which liquids may be miscible or immiscible with water. A raw or waste water treatment process, as that terminology is meant herein and as that terminology is understood in the raw and waste water treatment field, is a process in which a raw or waste water stream and/or system is treated to separate the raw or waste water stream and/or system into a plurality of fractions, one of which fractions having a higher concentration of at least one of the initial components. The separation technique(s) most commonly employed in raw and waste water treatment processes are one or more of solute/solid conversions, liquid/liquid separations, gas/liquid separations, solid/liquid separations, although gas/gas separations and solid/solid separations and other more refined techniques are at times encountered. When a raw or waste water stream and/or system contains solids, the initial waste water treatment techniques employed are very often that of bulk solid/liquid separation, for instance by filtration (gravity or vacuum assisted, air flotation-assisted, screening, straining belt-pressing and the like), settling, clarification or centrifugation, or combinations thereof, and such bulk separation techniques may be enhanced by the use of coagulants, flocculants and other solids-concentrating active chemicals. The water phase may be concentrated or treated (for instance by a pH adjustment) to decrease the dissolved solids content thereof or release emulsified components by means of emulsion breaking, before or after such bulk solids separation techniques are applied. The solids may be further dewatered. And as noted above, in some raw and waste water treatment processes dissolved or entrained gases may be separated from the liquid, liquids may be fractionated, solids may be fractionated, and so forth. Also as noted above, the primary feature of raw and waste water treatment processes is that a water stream and/or system having a plurality of components (including the water thereof) is separated into a plurality of fractions, and at least one of those fractions has a higher concentration of at least one of such components than the original water stream and/or system. When the technique employed is one of the solids/liquid separation techniques, the resultant fractions typically include one having a high concentration of solids, and one having a low concentration of solids and thus a higher concentration of water.

The ultimate fractions produced in a typical waste water treatment process are destined either for recycle to the process generating the waste or to a different process, or for disposal. These "products" of a waste water treatment plant seldom have a value commensurate with that of the process generating the waste. A typical waste water treatment plant is therefore extremely sensitive to the economics of the treatment process involved, and unlike the average process plant, its influent is extremely variable and its mechanical monitoring means are far less sensitive than that of a process plant. The monitoring of the hydraulic characteristics of a waste water treatment process is extremely important because such monitoring permits some degree of control of such hydraulic characteristics, which is often the primary process control means to a waste water treatment plant. The process of the present invention enables a plant to be proactive in its handling of mechanical problems and inlet water quality upsets by providing data in a trendable form. Conventionally the response to mechanical problems and inlet water quality upsets has been a reactionary or crises type of response.

Raw water streams and/or systems for the purposes of the present invention are waters being prepared for addition to and use in a process or cooling water system or boiler stream, and include without limitation well water, river water and other surface water supplies. Waste waters and/or systems for purposes of the present invention are most often waters that have been discharged from a process or cooling water system or boiler stream. (Some sewer waters are waters that have been discharged from a process, while other sewer waters are not and yet are within the category of waste waters for the purposes of the present invention.)

Both raw and waste water treatment processes are clarification or waste reduction processes by which the water is to some degree purified.

The primary differences between industrial raw and waste water streams and/or systems and other adjuvant industrial water streams and/or systems, such as boiler and cooling water streams and/or systems, are the concentrations of organic compounds and/or heavy metal contaminants. Concentrations of organic compounds and/or heavy metal contaminants in industrial boiler would not exceed about 5 ppm because such boiler and cooling water streams and/or systems cannot tolerate levels of 5 ppm or more of organic compounds and/or heavy metal contaminants. (This intolerance for organic compounds and/or heavy metal contaminants is one of the reasons that raw waters intended for use in a boiler and cooling water streams and/or systems are first subjected to a water treatment process.) In contrast, the concentration of organic compounds and/or heavy metal contaminants in the waters fed to industrial raw and waste water treatment processes will invariably have concentrations of organic compounds and/or heavy metal contaminants exceeding 5 ppm, and routinely such concentrations will exceed 10 ppm.

The elapsed time between the addition of the fluorescent specie(s) and the sampling to determine its presence and optionally its concentration at a remote site, or to determine the change in its concentration at or about the addition site, depends in part on the location of the available or desirable sample points. Generally the elapsed time between the addition of the fluorescent specie(s) and the sampling can be as short as about one minute or even less, and as long as about 24 hours or more.

The raw and waste water streams and/or systems of typical raw and waste water treatment plants can be characterized by the following physical and/or chemical property ranges: a pH of from about 4 to about 11; a temperature of from about 10 °C (50 °F) to about 45 °C (113 °F); an insoluble solids content of from about 20 ppm to about 1,000 ppm; a total solids content of from about 100 ppm to about 5,000 ppm. A difference in physical and/or chemical properties of the inlet water stream that would considered sufficiently abnormal or harmful so as to be an "upset" will of course vary from plant to plant.

The fluorescent specie(s) is herein at times referred to as a "tracer", the functional "tracer" terminology being applicable to the fluorescent specie(s) because it acts as a tracer for the hydraulic monitoring purposes of the present invention. The analytical techniques usable for tracking the fluorescent specie(s) include, but are not limited to, singular, intermittent, semi-continuous and continuous fluorescence analysis, including the combination of high pressure liquid chromatography ("HPLC") and fluorescence emission spectroscopy.

### Fluorescence Emission Spectroscopy

The detection and quantification of specific substances by fluorescence emission spectroscopy is founded upon the proportionality between the amount of emitted light and the amount of a fluoresced substance present. When energy in the form of light, including ultra violet and visible light, is directed into a sample cell, fluorescent substances therein will absorb the energy and then emit that energy as light having a longer wavelength than the absorbed light. The amount of emitted light is determined by a photodetector. The present invention generally employs externally applied excitation energy (generally within the ultra violet wavelength region) and optical apparatus to aid the detection and measurement of the emission energy. In preferred embodiment, the light is directed into the sample cell through an optical light filter so that the light transmitted to the sample is of a known wavelength, which is referred to as the excitation wavelength and generally is reported in nanometers ("nm"). The emitted light is similarly screened through a filter so that the amount of emitted light measured is that at a known wavelength or a spectrum of wavelengths, which is referred to as the emission wavelength and generally also is reported in nanometers. When the measurement of a single fluorescent specie or a plurality of fluorescent species at low concentrations is desired or required, such as often is the case for the process of the present invention, the filters are set for a specific combination of excitation and emission wavelengths, selected for substantially optimum low-level measurements. Although many fluorescent substances absorb light in the visible wavelength region, as noted elsewhere herein, for purposes of the present invention the use of a fluorescent specie(s) that absorbs light in the visible wavelength region and thus colors a raw and/or waste water stream and/or system is excluded.

In general, the concentration of a fluorescent specie(s) can be determined from a comparison of a sample's emissions intensity to a calibration curve of the given fluorescent specie(s)'s concentration versus emissions, for the same set of excitation wavelength/emission wavelengths. Such a concentration-by-comparison method by which the sensed emissions are converted to a concentration equivalent preferably is employed to determine concentrations of a fluorescent specie(s) that are within the concentration range over which a linear emission response is observed, and this concentration range is referred to herein as the "linear-emission-response concentration range". The linear-emission-response concentration range is to some extent dependent upon the specific fluorescent specie(s) and the excitation wavelength/emission wavelength set employed. At fluorescent specie(s) concentrations higher than a given fluorescent specie(s)'s linear-emission-response concentration range, there is a negative deviation from ideal (linear) behavior, the degree of emission for a given concentration being less than predicted by a linear extrapolation. In such instances, a correction can be applied to determine the concentration of the fluorescent specie(s) in the sample directly from the calibration curve, or the sample can be diluted by known factors until the concentration of the fluorescent specie(s) therein falls within the linear-emission-response concentration range. If the fluorescent specie(s) is present in the sample at only very low concentrations, there are techniques for concentrating the fluorescent specie(s) by known factors until its concentration falls within the linear-emission-response concentration range or is otherwise more readily measured, for instance by liquid-liquid extraction. Nonetheless, preferably a calibration curve over the linear-emission-response concentration range would be prepared or obtained before employing a given fluorescent specie(s), and preferably the fluorescent specie(s) would be added to the water of the raw and/or waste water stream and/or system in an amount sufficient to provide a concentration of the fluorescent specie(s) at the sampling point that is within the linear-emission-response concentration range. Generally the linear-emission-response concentration range of a fluorescent specie(s) is sufficiently broad to readily estimate the amount of the fluorescent specie(s) that will be sufficient for this purpose. A linear-emission-response concentration range will most often extend through a concentration range from a concentration of "m" to a concentration of at least 3m.

A determination of the presence of a fluorescent specie(s) and often the concentration thereof in a sample taken from a raw or waste water stream and/or system of a raw or waste water treatment process generally can be made when the concentration of the fluorescent specie(s) in the sample is only several parts per million (ppm) or even parts per billion (ppb) or parts per trillion (ppt) for some of the fluorescent specie(s) that can be employed in the process of the present invention. In preferred embodiment, the amount of fluorescent specie(s) added to the raw or waste water stream and/or system should be sufficient to provide a concentration of the fluorescent specie(s) in the sample to be analyzed of from about 5 ppb to about 100 ppm. Such analyses, that is, the measurements of the light emitted in response to the light transmitted to the sample, can be made on-site, preferably on an almost instant basis or on a semi-continuous or continuous basis, with simple portable equipment, such as the photodetector and screens described above.

At times it is desired to employ a plurality of fluorescent specie(s). For instance, it may be desired to determine the flow rates of more than one raw or waste water stream which eventually combine and form a single stream, and to accomplish the sampling for each at about the same point downstream of the site(s) at which the streams combine. In such instance, a separate fluorescent specie may be used for each stream and added upstream of the first site of stream combination. Such separate and distinct fluorescent species can each be detected and quantified in a single raw or waste water sample if their respective wavelengths of emission do not interfere with one another. Thus concurrent analyses for multiple fluorescent species is possible by selection of fluorescent species having appropriate spectral characteristics. Preferably widely separated wavelengths of radiation should be used to excite each of the fluorescent species and their fluorescent emissions should be observed and measured at widely separated emission wavelengths, and a separate concentration calibration curve prepared or obtained for each fluorescent specie. In other words, more than one chemical fluorescent specie(s) can be employed, and then the presence and/or concentration of each such fluorescent specie(s) in the fluid system should be determined using analytical parameters (particularly the excitation/emission wavelengths) effective for each such fluorescent specie(s), which analytical parameters preferably are sufficiently distinct to differentiate between measurements.

Fluorescence emission spectroscopy on a substantially continuous basis, at least over an hour's time period, is one of the preferred analytical techniques for the process of the present invention.

One method for the continuous on-stream monitoring of chemical tracers by fluorescence emission spectroscopy and other analysis methods is described in U.S. Patent No. 4,992,380, B. E. Moriarity, J. J. Hickey, W. H. Hoy, J. E. Hoots and D. A. Johnson, issued February 12, 1991, incorporated hereinto by reference.

### Combined HPLC-Fluorescence Analysis

The combination of high-pressure liquid chromatography ("HPLC") and fluorescence analyses of fluorescent tracers is a powerful tool for the present monitoring process, particularly when very low levels of the fluorescent tracer are used or the background fluorescence encountered would otherwise interfere with the efficacy of the fluorescence analysis. The HPLC-fluorescence analysis method allows the tracer compound to be separated from the fluid matrix and then the tracer concentration can be measured. The combination of HPLC-fluorescence analysis is particularly effective for measuring minute levels of tracer in highly contaminated fluids.

In preferred embodiment, the combination of the chemical compound(s) selected as the fluorescent specie(s) and the analytical techniques or parameters selected for determining the presence and/or concentration of such fluorescent specie(s), should permit such determination(s) without isolation of the fluorescent specie(s), and more preferably should permit such determination(s) on an instant or continuous and/or on-line basis. Analytical techniques for detecting the presence and/or concentration of a chemical specie(s) without isolation thereof are within an evolving technology, and the above survey of reasonable analytical techniques for use in the process of the present invention may presently not even be exhaustive, and most likely techniques equivalent to the above for the purposes of the present invention will be developed in the future.

### The Fluorescent Specie(s)

As noted above, the fluorescent specie(s) of the present invention is one that does not strongly absorb in the visible light region, and hence is one that has a maximum excitation wavelength of less than about 400 nm, or in the section of the electromagnetic wavelength spectrum below (having wavelengths less than) visible light.

The fluorescent specie(s) should be soluble in the fluid of the raw and/or waste water stream and/or system, and since such fluid is an aqueous-based fluid (water or a mixture of water and a liquid miscible in water as the continuous phase), in preferred embodiments the fluorescent specie(s) should be water-soluble at least at the concentration of the fluorescent specie(s) that is employed in the present invention.

As noted elsewhere, the fluorescent specie(s) used in the present invention is preferably an inert chemical, not substantially consumed in the use environment. A fluorescent specie(s) that is wholly inert in the use environment would be a fluorescent specie(s) that does not react with any of the components in the raw or waste water stream and/or system to which it is added, does not degrade in the environment of the raw or waste water stream and/or system and is incapable of coupling and/or depositing in any manner within such raw or waste water stream and/or system. There are many water-soluble fluorescent specie(s) that are wholly inert, or almost wholly inert, in the aqueous environments likely to be encountered in raw or waste water treatment plants. Further, it is believed that a fluorescent specie(s) having a degree of inertness such that no more than 10 weight percent thereof is lost due to reaction, degradation, coupling and/or deposition during the time that elapses between addition and sampling is sufficiently inert for the purpose of the present invention for most, if not all, hydraulic characteristic monitorings. In preferred embodiment, the chemical compound(s) selected as a fluorescent specie(s) should not be one that is consumed or lost to the fluid, for instance due to degradation, deposition, complexation, or other phenomena, unless such consumption or loss is at a rate that is predictable and proportional to hydraulic characteristic being monitored.

Among these substantially inert fluorescent compounds are the mono-, di- and trisulfonated naphthalenes, particularly the various naphthalene sulfonic acid isomers, which are a preferred fluorescent specie(s) for use in the present invention. The naphthalene sulfonic acid isomers, particularly the water-soluble salts of naphthalene disulfonic acid ("NDSA"), for instance 1,5-NDSA, are water-soluble, generally available commercially and easily detectable and quantifiable by known fluorescence analysis techniques. Preferred concentrations of NDSA in the sample being analyzed are within the range of from about 0.1 to about 5 ppm. Many of these fluorescent specie(s) (mono-, di- and trisulfonated naphthalenes and mixtures thereof) are believed to be extremely compatible with the environments of most industrial raw or waste water streams and/or systems.

Another group of inert fluorescent compounds that are preferred for use in the process of the present invention are the various sulfonated derivatives of pyrene, such as 1, 3, 6, 8-pyrene tetrasulfonic acid, and the various water-soluble salts of such sulfonated pyrene derivatives.

Certain compounds are inherently fluorescent or may be tagged to provide a traceable fluorescent characteristic. Some naturally fluorescent compounds are also water treatment agents, and thus may be among the compounds normally added to a raw or waste water stream and/or system, for instance aromatic organic corrosion inhibitors, such as aromatic(thio)(tri)azoles. Some water treatment agents may be susceptible to tagging with fluorescent groups, for instance as disclosed in U.S. Patent No. 5,128,419, D. W. Fong and J. E. Hoots, issued July 7, 1992, incorporated herein by reference, wherein the tagging of polymers with pendant fluorescent groups by (trans)amidation derivatization of pre-existing polymers having carbonyl-type pendant groups is disclosed. Water-treatment polymers tagged with pendant fluorescent groups may of course be prepared by methods other than (trans)amidation derivatization. The polymers become themselves fluorescent, or more highly fluorescent, by virtue of appending thereto fluorescent groups, and some, but not all, of these derivatized polymers have their major absorption peaks at wavelengths below the visible light region of the electromagnetic radiation spectrum (less than about 400 nm). Such naturally fluorescent or fluorescence-tagged water-treatment compounds are useful for the purposes of the present invention only when they can provide the hydraulic characteristic monitoring desired by the present invention despite their potential for consumption to some degree in the performance of their water-treatment functionality(ies), and in preferred embodiment the use of fluorescent water-treatment specie(s), or other water-treatment specie(s), as the fluorescent specie(s) of the present process is excluded.

Other fluorescent chemical tracers and monitoring techniques are now known, for instance as disclosed in U.S. Patent No. 4,783,314, J. E. Hoots and B. E. Hunt, issued November 8, 1988, incorporated herein by reference, wherein inert fluorescent tracers are employed in combination with a fluorescence monitoring, such as the sodium salt of 2-naphthalenesulfonic acid ("NSA"). The Acid Yellow 7 dye also illustrated therein would generally be a visible dye, imparting coloration to a raw or waste water stream and/or system and absorbing strongly in the visible light region of the electromagnetic radiation spectrum, and thus not be suitable for purposes of the present invention.

In general, for most fluorescence emission spectroscopy methods having a high degree of practicality, it is preferable to perform the analysis without isolating in any manner the fluorescent specie(s). Thus there may be some degree of background fluorescence in the raw or waste water sample in which its emission is observed, and its concentration determined. In instances where the background fluorescence is low, the relative intensities (measured against a standard fluorescent compound at a standard concentration and assigned a relative intensity, for instance of 100) of the fluorescence of the fluorescent specie(s) versus the background can be very high, for instance a ratio of 100/10 or 500/10 when certain combinations of excitation and emission wavelengths are employed even at low fluorescent compound concentrations, and such ratios would be representative of relative performance (under like conditions) of respectively 10 and 50. For most raw or waste water treatment backgrounds, a compound that has a relative performance of at least about 5 at a reasonable concentration is very suitable as a fluorescent specie(s) itself or as a tagging agent for water treatment polymers and the like when such compounds contain an appropriate reactive group for the tagging reaction. When there is, or may be, a specific chemical specie(s) of reasonably high fluorescence in the background, the fluorescent specie(s) and the excitation and/or emission wavelengths often can be selected to nullify or at least minimize any interference from light emitted by the background chemical, or alternatively the amount of fluorescent specie(s) added to the raw or waste water stream and/or system can be increased to substantially overcome the interference from background fluorescence.

In preferred embodiment the fluorescent specie(s) is selected from those that have a reasonably high "relative fluorescence" and/or "relative performance" for a given background. A molecule is generally considered highly fluorescent when it has a fluorescent quantum efficiency, or fluorescent quantum yield, within the range of from about 0.1 to about 1.0, and a light absorbance molar extinction coefficient of at least 1,000. The magnitude of fluorescent signal, or "relative fluorescence", is the product of the fluorescent quantum yield multiplied by the light absorbance molar extinction coefficient. Thus for instance if two compositions each had the minimum 1,000 light absorbance molar extinction coefficient, but one had a fluorescent quantum yield of 0.1 and the other had a fluorescent quantum yield of almost 1.0, the latter would have a ten-fold higher relative fluorescence. The "relative performance", as this terminology is used herein, is the ratio of the relative fluorescence of a fluorescent specie(s) to the relative fluorescence of a given background medium, both when determined using a given set of excitation and emission wavelengths. That set of excitation and emission wavelengths generally would be selected to coincide with, or be reasonably close to, the wavelengths of absorption and emission at major peaks of the given fluorescent specie(s), which commonly would be reasonably separated from the wavelengths of maximum absorption and emission of the given background, and this factor alone would diminish the background's relative performance and provide a reasonably high ratio. Nonetheless, two fluorescent species may have similar relative fluorescence values, but different major-peak excitation and/or emission wavelengths, and therefore have diverse relative performance values, the fluorescence intensity of the background differing at these diverse wavelengths. Since fluorescence intensity increases with increasing concentration of a fluorescent specie(s), as mentioned elsewhere herein, interference from background fluorescence can be diminished by increasing the concentration of the fluorescent specie(s) added to the raw or waste water stream and/or system.

In general, however, if there is a discernable difference between the fluorescence of the background alone and that of the background and tracer together at the sample point(s), the tracer chosen has a sufficiently high relative performance versus the background for purposes of the present invention.

A fluorescent specie(s) may be selected for a given process based on a preference for one or more analytical techniques or parameters, or the analytical techniques or parameters may be selected for a given process based on a preference for one or more fluorescent specie(s). In preferred embodiment, the chemical compound(s) selected as the fluorescent specie(s) should be soluble in the fluid of the process, at least at the concentration level(s) of the chemical fluorescent specie(s) employed in the respective fluid. In one preferred embodiment, the chemical compound(s) selected as a fluorescent specie(s) should be either substantially inert in the environment of the raw or waste water stream and/or system for the useful life expected of the fluorescent specie(s), or its loss from the raw or waste water stream and/or system due to degradation, deposition, complexation, or other phenomena should be predictable and compensative, particularly when it is desired not merely to detect the presence of some amount of the fluorescent specie(s), but also to determine the concentration thereof, or change in concentration.

In another preferred embodiment, the analytical technique(s) or parameters selected for determining the presence and/or concentration of a fluorescent specie(s), should permit such determination(s) to provide a signal that can activate or regulate the one or more of the hydraulic systems of the raw or waste water treatment plant to provide an appropriate hydraulic system modification in response to the hydraulic characteristic monitoring.

In one embodiment, the addition point and sampling point for the fluorescent specie(s) are either both upstream of, or downstream of, any flow-though site having a high concentration of solids, for instance a solids concentration of at least about 5 or about 10 weight percent per unit volume, wherein the volume unit measured is about one cubic inch. Such high solids concentration flow-through sites are found at the site of filter cakes and the like, and may absorb at least some amount of even an inert fluorescent specie(s), distorting the hydraulic characteristic monitoring. By adding and sampling either upstream or downstream of such sites, the need to predetermine whether, and to what extent, a fluorescent specie(s) is absorbed by such solids when present at such concentrations is avoided.

Other sites that at some times may also be avoided, that is not permitted to be interposed between the addition of the fluorescent specie(s) and the water sampling for its measurement, are sites of solids concentration(s) using enhancing chemical additives such as coagulants, flocculants and the like.

### Example 1

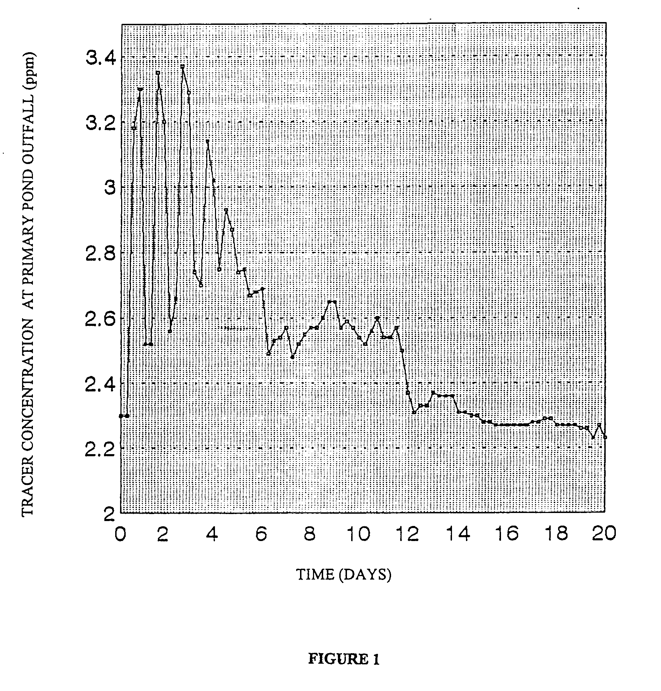

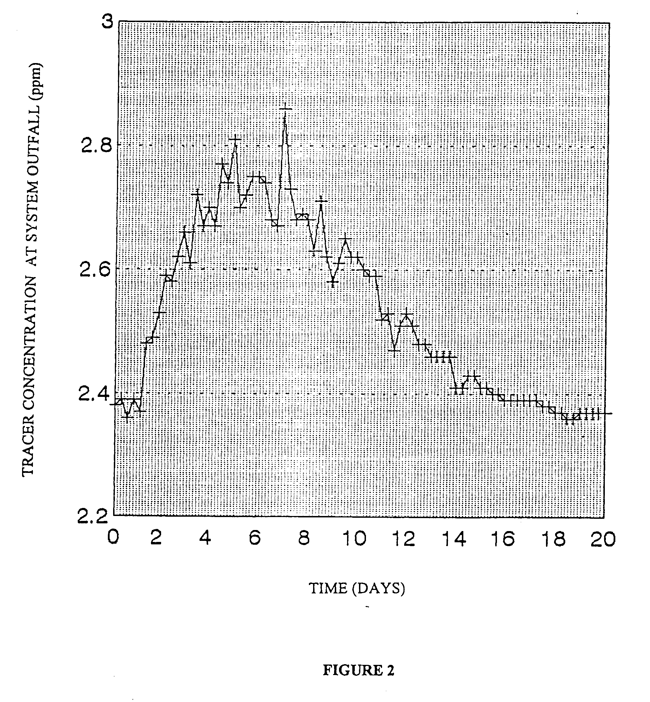

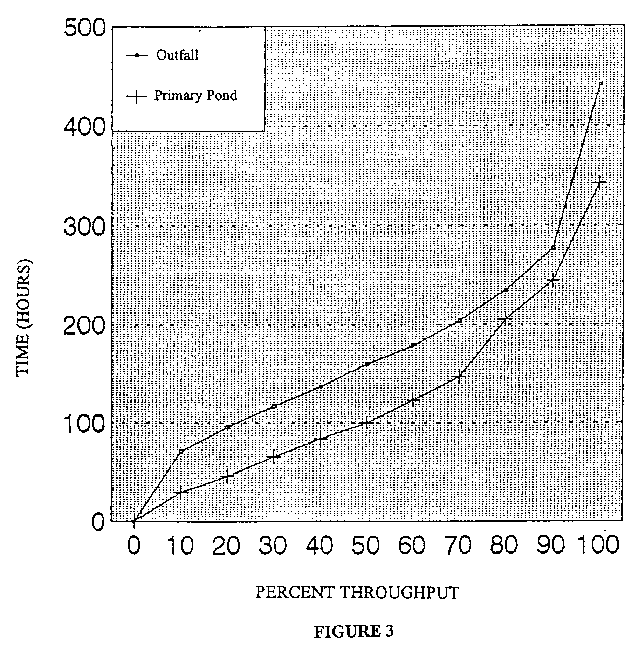

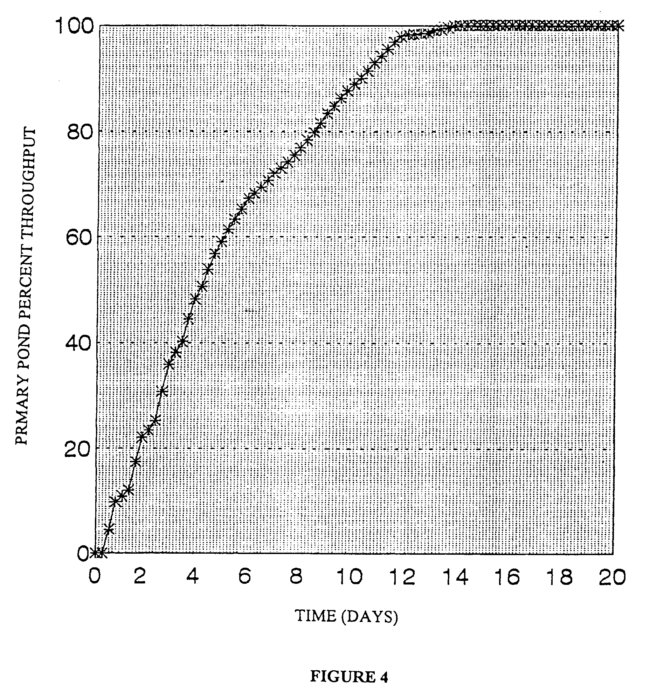

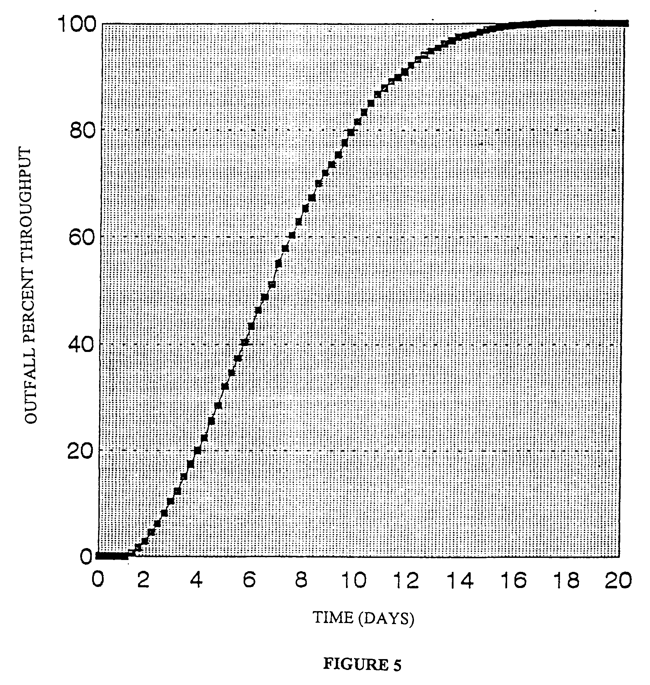

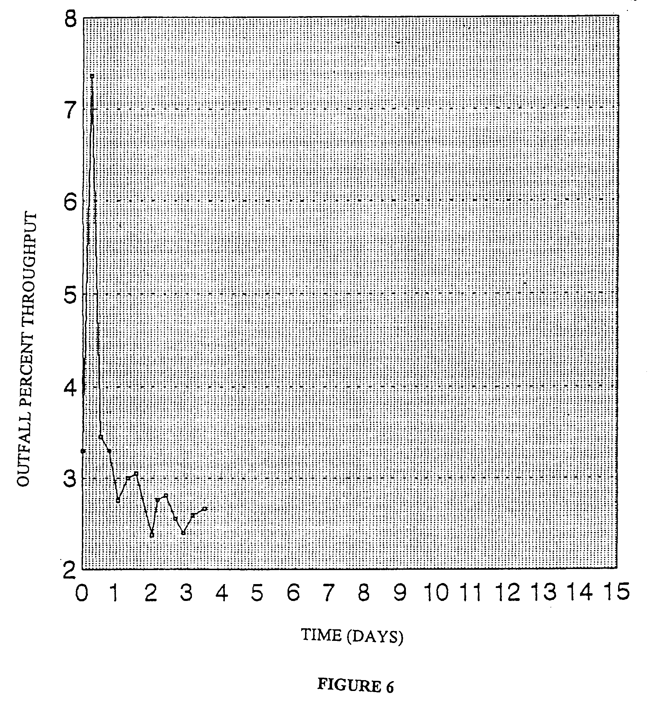

An industrial water system had been experiencing inlet low pH excursions that diminished the efficiency of the waste water treatment and thus caused problems in permit compliance. Such treatment problems and any other inlet water upsets could be handled more practically if the duration of such upset and the time of maximum effect from such upset and were known. The retention time of a water-soluble specie from the point of inlet to the treatment system to the point of discharge from the treatment system was determined as follows. Samples of the water at the outfall and the inlet to the primary and secondary ash basins were collected and analyzed for fluorometric emissions using a standard set of excitation and emission wavelengths. The standard excitation and emission wavelengths were selected based on the fluorescent specie (tracer) chosen for use in the study. Such analyses provide "baseline" conditions or background fluorescence levels, which were converted to their equivalent in terms of concentrations of the tracer. Such background fluorescence determinations ranged from 2.3 to 2.5 ppm of tracer, which levels of background fluorescence were acceptably low for conducting the study with the chosen tracer. These outfall and inlet samples were then each spiked with a small amount of the tracer, reanalyzed fluorometrically, and then allowed to sit for 48 hours to ensure that the chosen tracer did not degrade in these individual water quality and contaminant environments. The samples were then again analyzed fluorometrically and there were no appreciable differences in the fluorometric readings before and after the 48 hour time period. The system was then spiked with the tracer at the inlet to the ash ponds and thereafter samples were collected at four-hour intervals at each of the primary-pond inlet, the secondary-pond inlet and the outfall. After two weeks of sample collection, the samples were each analyzed fluorometrically using an on-site lab-top fluorometer to determine the apparent concentrations of the tracer in each. The sample collection was continued for an additional week at the secondary pond inlet and the outfall, after which those samples were also analyzed fluorometrically using an on-site portable fluorometer to determine the apparent concentrations of the tracer in each. All data was adjusted by subtracting the respective background fluorescence from the apparent concentration of the tracer reading to provide the more precise "adjusted" concentration reading. In FIGURE 1 is shown a plot of the apparent (uncorrected) concentrations of the tracer at the primary-pond outfall versus time (in days). In FIGURE 2 is shown a plot of the apparent (uncorrected) concentrations of the tracer at the system outfall versus time (in days). In FIGURE 3 is shown the plots of the percent throughput versus time (in hours) between the primary-pond inlet and primary-pond outfall (designated "Primary Pond") and then between the primary-pond inlet and system outfall (designated "Outfall") using the apparent (unadjusted) tracer concentration readings. In FIGURES 4 and 5 respectively are shown the plots of the percent throughput versus time (in days) between the primary-pond inlet and primary-pond outfall (designated "Primary Pond") and then between the primary-pond inlet and system outfall (designated "Outfall") using adjusted tracer concentration readings. In FIGURE 6 is shown a plot of the apparent tracer concentration versus time (in days) at the primary-pond inlet. Referring most particularly to the data shown in FIGURES 1 and 2, the time intervals of primary concern are as set forth in Table 1 below.

**Table 1**

| Time Interval | Location | |
|---|---|---|
| Time Elapse from Inlet Upset To The: | Primary Pond Outfall | System Outfall |
| First Significant Upset Effect | 1.2 days | 3.0 days |
| Maximum Upset Effect | 4.2 days | 6.7 days |
| End Of Significant Upset Effect | 9 days | 14 days |

As seen from Table 1 above, the hydraulic characteristics monitored and determined in this study were far more complicated than a mere flow rate from the inlet to the primary-pond outfall and from the primary-pond outfall to the system outfall. Additionally, as seen from FIGURES 1 and 4, the throughput through the primary pond was cyclic during the first four to five days of the study. The primary pond samples collected during the daylight hours had high concentrations of the tracer, while the samples collected during the night hours had low concentrations of the tracer. The throughput versus time curve for the system outfall, FIGURE 5, is nonetheless very close to a traditional bell-shaped curve. The study therefore also revealed the hydraulic characteristics of the night-time turning over and the day-time short circuiting of the primary pond while such phenomena were not occurring in the secondary ponds, ameliorating the effect upon the overall water system. This study provided data usable in the future to plan and implement how and when to add treatments and/or make flow adjustments to prevent exceeding treatment process control parameters and outfall permit limits. The data also allows the plant to recognize when such actions should be terminated because no further positive control value will be provided therefrom.

In certain embodiments of the invention, the process includes the preliminary step of sampling the raw or waste water stream and/or system to determine baseline conditions, preferably at all intended or possible post-fluorescent specie(s)-addition sampling sites. Such preliminary baseline condition(s) determination(s) may also be employed to narrow the selection choice of fluorescent specie(s) or determine and/or modify the selection of post-fluorescent specie(s)-addition sampling points.

At times it may be most efficient to monitor the route that is most sensitive to both the detection and quantification of the fluorescent specie(s) chemical, which sensitivity may be derived from the baseline condition(s) of such route or the concentration of fluorescent specie(s) chemical expected along such route. Monitoring the most sensitive route or the route of most concern in a given industrial raw or waste water treatment plant may not, however, be the most practical. For instance, the water line(s) of such route(s) may be less accessible to monitoring than other water lines. Thus while the monitoring of the water within the route of most concern or the route most sensitive to monitoring is desirable, circumstances can render that approach less desirable for practical reasons.

Generally it is desirable to employ the least amount of fluorescent specie(s) that is practical for the circumstance, and the amount of the fluorescent specie(s) added to the raw or waste water stream and/or system should be at least an amount effective for the determinations desired. Seldom would a fluorescent specie(s) be deliberately fed to a raw or waste water stream and/or system in an amount grossly in excess of the minimum effective amount because there generally would be no practical purpose in doing so that would justify the costs involved. The amount of fluorescent specie(s) to be added to the raw or waste water stream and/or system that is effective without being grossly excessive will vary with a wide variety of factors, including without limitation the fluorescent specie(s) and analysis parameters selected, the potential for background interference with the selected monitoring method, and the monitoring mode (on-line continuous, semi-continuous, slug-and-sample, and the like). Generally the dosage of fluorescent specie(s) added to the raw or waste water stream and/or system will be at least sufficient to provide a concentration of fluorescent specie(s) therein of about 0.005 ppm, and more commonly at least about 10 ppm or higher.

The present invention in an embodiment is a process for monitoring at least one hydraulic characteristic in a raw or waste water stream and/or system of a raw or waste water treatment process, comprising: adding to the raw or waste water stream and/or system at least one fluorescent specie(s); and subjecting at least one sample of water from at least one site of the raw or waste water stream and/or system to at least one analysis; wherein the analysis at least detects the presence of the fluorescent specie(s) in the sample, and wherein the analysis at least determines one hydraulic characteristic of the raw or waste water treatment process by a comparison between such determination and a known relationship between the presence and/or concentration of the fluorescent specie(s) and such hydraulic characteristic.

The present invention in an embodiment is a process for monitoring at least one hydraulic characteristic of a raw or waste water stream and/or system of a raw or waste water treatment process, comprising: adding to the raw or waste water stream and/or system of a raw or waste water treatment process at least one fluorescent specie(s), wherein the relationship between the presence and/or concentration of the fluorescent specie(s) at a given site and at least one hydraulic characteristic of the process is substantially known; subjecting at least one sample of water from at least one site of the raw or waste water stream and/or system of a raw or waste water treatment process to at least one analysis; and wherein the analysis detects the presence and/or concentration of the fluorescent specie(s) in the sample, and then determining at least one hydraulic characteristic of the process by a comparison between such determination and a prior determination of the presence and/or concentration of the fluorescent specie(s) at at least one site in the aqueous system.

The present invention in an embodiment is a raw or waste water treatment process comprising: adding at least one fluorescent specie(s) to the water in the aqueous system of a raw or waste water treatment process; determining at at least one site of the aqueous system of the raw or waste water treatment process the presence and/or concentration of the fluorescent specie(s); and then comparing such determination to the presence and/or concentration of the fluorescent specie(s) at at least one other site in the aqueous system.

In preferred embodiments, the analysis determines the concentration of the fluorescent specie(s) in the raw or waste water stream and/or system of the raw or waste water treatment process. The specie of chemical fluorescent specie(s) in some preferred embodiments is a sulfonated naphthalene (particularly one of the disulfonated naphthalene isomers) or a sulfonated pyrene or combinations thereof.

The analysis at least includes fluorescence analysis and may be an intermittent, on-line continuous or semi-continuous analysis. At times at least a detection of the fluorescent specie(s) activates or modifies a signal, and the signal upon activation or modification activates or modifies a hydraulic characteristic of the raw or waste water treatment process.

In certain preferred embodiments, the fluorescent specie(s) is foreign to the normal treatment program for the raw or waste water stream and/or system of a raw or waste water treatment process, for instance when an inert fluorescent specie(s) is added for the purposes of present invention. In certain preferred embodiments, the fluorescent specie(s) is substantially resistant to depletion mechanisms in the environment of the water, such as degradation, deposition, consumption and other like depletion mechanisms. In certain embodiments, the fluorescent specie(s) is substantially susceptible to depletion mechanisms in the environments of the raw or waste water stream and/or system of a raw or waste water treatment process in a degree or at a rate that is in proportion to at least one hydraulic characteristic.

The process of the present invention may monitor one of any of the hydraulic characteristic of a raw or waste water stream and/or system, or may simultaneously monitor a plurality of hydraulic characteristics of a raw or waste water stream and/or system.

Unless expressly indicated otherwise herein, all properties of any chemical compounds, or compositions containing a plurality of chemical compounds, set forth herein are such property values as would be determined for such compounds, or compositions, within a temperature range of from about 20 °C to about 40 °C, and substantially under atmospheric pressure.

By deposits is meant herein material that forms and/or collects on surfaces in contact with a raw or waste water stream and/or system of a raw or waste water treatment process. By the terminology "detection of the presence of" a chemical species is meant herein the determination of whether or not such chemical species is present or absent, and thus includes the detection of the absence of such chemical species, at least to the limitations of the analytical method employed. By raw or waste water stream(s) and/or system(s) is meant water stream(s) that are alone a water system or comprise a water system with other bodies of water and/or water systems that are comprised of of at least one water stream or that include at least one water stream together with at least one other body of water or are comprised of one or more body of water that is sufficiently nonflowing to be considered a body of water other than a stream. By "throughput time" is meant herein the time in which a specie(s) is substantially totally removed from a stream and/or system measured against the time of its introduction to the stream and/or system. By "channelling" is meant herein the creation or existence of a preferential flow route in a stream and/or system. By "rise rate" is meant the upward movement of a certain fraction of a stream and/or system and the stream and/or system.

Unless expressly indicated otherwise herein, the inclusion of a prefix or suffix in parenthesis designates the word with such prefix or suffix as an alternative. For instance, "specie(s)" means "specie and/or species", "determination(s)" means "determination and/or determinations", "characteristic(s)" means "characteristic and/or characteristics", "technique(s)" means "technique and/or techniques", "location(s)" means "location and/or locations", "chemical(s)" means "chemical and/or chemicals", "component(s)" means "component and/or components", "functionality(ies)" means "functionality and/or functionalities", "aromatic(thio)(tri)azoles" means aromatic azoles and/or aromatic thiazoles and/or aromatic triazoles", and the like.

### Industrial Applicability of the Invention

The present invention is applicable to all industries employing a raw or waste water stream and/or system of a raw or waste water treatment process that has at least one hydraulic characteristic.

## Claims

1. A process of monitoring a characteristic of a raw or waste water treatment process which characteristic is effected by the movement of water within the raw or waste treatment process comprising:
adding to said raw or waste water treatment process at least one soluble, substantially inert fluorescent specie(s) having a maximum excitation wavelength of less than about 400 nm; and
subjecting at least one sample of water from at least one site of said raw of waste water treatment process to at least one analysis;
wherein said analysis at least detects the presence of said fluorescent species(s) in said sample, and
wherein said analysis determines at least one characteristic of said raw or waste water treatment process by a comparison between said analysis and a known relationship between the presence and/or concentration of said fluorescent specie(s) and said characteristic.

2. The process of claim 1 wherein said characteristic is the throughput time of a water-soluble specie(s) between the point of addition of said fluorescent specie(s) and the point of taking said sample of water.

3. The process of claim 1 or 2 wherein said addition of said fluorescent specie(s) is on a substantially continuous basis, a plurality of said analysis determines the change in the concentration of said fluorescent specie(s), and optionally said determined characteristic is then adjusted.

4. The process of any of claims 1 to 3 wherein said analysis is an intermittent analysis or an instant, on-line continuous or semi-continuous analysis.

5. The process of any of claims 1 to 4 wherein said addition of said fluorescent specie(s) is the addition of a slug of said fluorescent specie(s) at a known point in time and said monitoring is comprised of analysis of a plurality of samples taken at known times from at least one site downstream of the site of said addition.

6. The process of any of claims 1 to 5 wherein said monitoring is comprised of tracking the rise and drop in the concentration of said fluorescent specie(s) at at least one site downstream of the addition point of said fluorescent specie(s) and optionally controlling said characteristic being determined by said monitoring.

7. The process of any of claims 1 to 6 wherein said analysis determines the concentration of said fluorescent specie(s).

8. The process of any of claims 1 to 7 wherein said raw or waste water treatment process contains at least about 10 ppm organic compounds and/or heavy metal contaminants at the point said raw or waste water enters said raw or waste water treatment process.

9. The process of any of claims 1 to 8 wherein said fluorescent specie(s) is at least one of a naphthalene sulfonic acid, a naphthalene disulfonic acid, a pyrene tetrasulfonic acid, a water-soluble salt of a naphthalene sulfonic acid, a water-soluble salt of a naphthalene disulfonic acid, a water-soluble salt of a pyrene tetrasulfonic acid, or combinations or mixtures thereof.

10. A raw or waste water treatment process of the type wherein a multi-component raw or waste water is treated to separate the raw or waste water into a plurality of fractions, whereby the concentration of at least one of the components of said raw or waste water is concentrated in at least one but less than all of said fractions the improvement comprising monitoring a characteristic effected by the movement of water within the multi-component raw or waste water by the steps comprising:
adding to said water at least one water soluble, substantially inert fluorescent specie(s) having a maximum excitation wavelength of less than about 400 nm; and
monitoring a characteristic of said raw or waste water treatment process by determining at at least one site of said raw or waste water treatment process the presence and/or concentration of said fluorescent specie(s); and
then comparing said determination to the presence and/or concentration of said fluorescent specie(s) at at least one other site of said raw or waste water treatment process.

11. The process of claim 10 wherein said specie of fluorescent specie(s) is substantially inert in the environment of said raw or waste water treatment process.

12. The process of claim 10 or 11 wherein said analysis determines the cencentration of said fluorescent specie(s).

13. The process of any of claims 10 to 12 wherein said addition of said fluorescent specie(s) is the addition of a slug of said chemical fluorescent specie(s) at a known point in time and said monitoring is comprised of analysis of a plurality of samples taken at known times from at least one site downstream of the site of said addition.

14. The process of any of claims 10 to 13 wherein a plurality of said fluorescent species are each added to said water treatment process at different sites of said water treatment process.

15. The process of claim 14 wherein said water treatment process includes a plurality of water streams and wherein said different sites are along different water streams.

16. The process of any of claims 10 to 15 wherein the fluorescent specie(s) is at least one of a naphthalene sulfonic acid, a naphthalene disulfonic acid, a pyrene tetrasulfonic acid, a water-soluble salt of a naphthalene sulfonic acid, a water-soluble salt of a naphthalene disulfonic acid, a water-soluble salt of a pyrene tetrasulfonic acid, or combinations or mixtures thereof.

17. The process of any of claims 1 to 16 wherein said characteristic effected by the improvement of water within the raw or waste treatment process is selected from the group consisting of throughput time, water stream route, channeling, rise rate, carryover, cross-contamination, leaks between systems, flow rate and flow path.
